Europäisches Patentamt

European Patent Office

Office européen des brevets

(19)

(11) Veröffentlichungsnummer: **0 320 433**
**A2**

(12) **EUROPÄISCHE PATENTANMELDUNG**

(21) Anmeldenummer: 88730268.5

(22) Anmeldetag: 02.12.88

(51) Int. Cl.⁴: **A 61 K 49/00**
**A 61 B 8/08**

(30) Priorität: 02.12.87 DE 3741201

(43) Veröffentlichungstag der Anmeldung:
14.06.89 Patentblatt 89/24

(84) Benannte Vertragsstaaten: **ES GR**

(71) Anmelder: **SCHERING AKTIENGESELLSCHAFT Berlin
und Bergkamen**
**Müllerstrasse 170/178 Postfach 65 03 11**
**D-1000 Berlin 65 (DE)**

(72) Erfinder: **Schlief, Reinhard, Dr.**
**Neue Strasse 21**
**D-1000 Berlin 37 (DE)**

(74) Vertreter: **Maikowski, Michael, Dipl.-Ing. Dr.**
**Xantener Strasse 10**
**D-1000 Berlin 15 (DE)**

(54) **Ultraschall- oder Stosswellenarbeitsverfahren und Mittel zu dessen Durchführung.**

(57) Die Erfindung betrifft ein Stoßwellen- oder Ultraschallar-beitsverfahren bei dem mittels Stoßwellen- oder Ultraschaller-zeugern Stoßwellen oder Ultraschall in einem Medium erzeugt werden, in dem eine Wirkung erzielt werden soll, wobei diesem Medium ein Mittel zugesetzt wird, welches Mikrobläschen enthält oder erzeugt, die eine Wirkungsverstärkung der Stoß-wellen oder des Ultraschalls herbeiführen und Mittel zur Durchführung des Verfahrens, wobei diese Mittel auch zur gezielten Zerstörung biologischer Gewebe mit Ultraschall und Stoßwellen verwendet werden können.

EP 0 320 433 A2

## Beschreibung

### Ultraschall- oder Stoßwellenarbeitsverfahren und Mittel zu dessen Durchführung

Die Erfindung betrifft ein Verfahren nach dem Oberbegriff des Patentanspruchs 1 und Mittel zur Durchführung des Verfahrens.

Ein wichtiges Anwendungsgebiet der Ultraschalltechnik ist die zerstörunsfreie Materialprüfung. H. O. Richter beschreibt in: Taschenbuch Akustik - Berlin: Verlag Technik 1984 die Ultraschallmaterialprüfung und in diesem Bericht wird die Kopplungstechnik, d. h. die Ankopplung des Ultraschallschwingers an den Prüfling erläutert.

In der Zeitschrift "ULTRASONICS" July 1985 S. 170- 172 wird ein Verfahren beschrieben, mit dem eine Glasplatte mittels eines Ultraschallfeldes in Wasser gereinigt wird. Im Wasser werden Luftblasen durch Ultraschall erzeugt, deren Durchmesser größer ist als der der Luftblasen, die durch die Grundfrequenz (25 kHz) des eingestrahlten Ultraschallfeldes erzeugt werden. Die erste Harmonische (47,2 kHz) des eingestrahlten Ultraschallfeldes wirkt als Oberflächenwelle auf die Grenzfläche Wasser/ Luft der Luftblasen ein und erzeugt Tochter-Mikro-Bubbles, deren Durchmesser kleiner ist als der der Mikro-Bubbles, die von der ersten Harmonischen allein erzeugt werden, Mit diesem Bläschensystem wurde eine Entfernung des Beschichtungsmaterials auf der Glasplatte durchgeführt. Bei diesem Verfahren müssen hochenergetische Ultraschallfelder eingestrahlt werden, die nicht für alle Materialien verträglich sind.

Der vorliegenden Erfindung liegt die Aufgabe zugrunde, zur Verbesserung der Ultraschallarbeitsverfahren ein spezielles Verfahren und Mittel zur Durchführung dieses Verfahrens zur Verfügung zu stellen, mit denen gute Ergebnisse mit geringerem Energieeinsatz als bei bekannten Verfahren und insbesondere unter Verwendung von Stoßwellen erzielbar sind.

Erfindungsgemäß wird diese Aufgabe durch die technische Lehre des Inhalts des Patentanspruchs 1 in Verbindung mit den angegebenen Mitteln gelöst.

Dem Medium, in dem die gewünschte Arbeit durchgeführt oder eine vorbestimmte Wirkung erzielt werden soll, werden die Mikrobläschen erzeugenden oder enthaltenden Mittel appliziert, wodurch die Wirkung eingestrahlter Stoßwellen oder Ultraschallfelder synergistisch verbessert wird. Gleiche Effekte werden mit geringer eingestrahlter Energie erreicht. Bei gleicher Energie ergeben sich größere Effekte.

Bei Ultraschalleinstrahlung ist es besonders vorteilhaft, die Mikrobläschengröße so einzustellen, daß deren Resonanzfrequenz im Frequenzbereich des Ultraschalls liegt.

Bei dem Arbeitsverfahren werden die Stoßwellen oder das Ultraschallfeld durch fokusierende Erzeuger in das Medium abgestrahlt, in welches die Mikro-Bubbles enthaltenden oder erzeugenden Mittel eingebracht werden.

Stoßwellen oder Ultraschallfelder werden in einen vorbestimmten Bereich mit Vorteil von Erzeugern aus unterschiedlichen Richtungen eingestrahlt. Es ist ferner vorgesehen, den oder die Erzeuger der Stoßwellen oder Ultraschallfelder um den Fokus herum zu verschwenken.

Von besonderer Bedeutung ist die verstärkende Wirkung bei Oberflächenbehandlungen von Werkstücken wie Oberflächenvergütung und dergl. mittels Stoßwellen oder Ultraschall. Ein weiteres Anwendungsgebiet ist die Stoßwellen- oder Ultraschallreinigung, bei der die Stoßwellen oder Ultraschallenergie über den Boden und über die Wände einer Reinigungswanne oder über eingehängte Erzeuger in die Reinigungsflüssigkeit abgestrahlt wird. Das Reinigungsgut kann in Körben oder Trommeln und bei großen Anlagen durch ein Transportsystem in die Reinigungsflüssigkeit gebracht werden. Durch den erfindungsgemäßen Einsatz des Mikrobläschen enthaltenden oder erzeugenden Mittels können die Reinigungszeiten ganz erheblich verkürzt werden und es werden außerordentlich porensaubere Oberflächen erzielt.

Das erfindungsgemäße Verfahren kann auch bei der Desintegration Anwendung finden, die den Einsatz von Stoßwellen oder Leistungsultraschall zur Extraktion, Dispergierung (Emulgierung, Homogenisierung) und für andere Prozesse unfaßt. Mit diesem Verfahren können Mikro-Organismen wie Bakterien, Viren und Pilze aufgeschlossen werden. Es können Inhaltsstoffe aus pflanzlichem und tierischem Material extrahiert werden. Schwer lösliche Substanzen können in Flüssigkeiten emulgiert werden und feste unlösliche Stoffe können in Feinstverteilung in Flüssigkeiten dispergiert werden. Ferner können chemische und biologische Reaktionen beschleunigt und Proben für mikroskopische und elektronenmikroskopische Untersuchungen aufbereitet werden.

Bei der Extraktion kann die Kavitation zu einer Zell-oder Stoffzerstörung und zur Dispersion der suspendierten Festkörper in Verbindung mit erhöhtem Massentransport durch eine akustische Strömung führen. Durch das erfindungsgemäße Verfahren werden erhöhte Extraktionsraten bei der Extraktion von Zucker aus Zuckerrüben, bei der Hopfenextraktion, bei der Proteinfällung, beim Bierbrauprozeß, bei der Ölgewinnung aus Ölsamen und aus Fisch und bei der Extraktion von Alkaloiden aus pflanzlichen Materialien und bei der Auslaugung von Erzen erzielt.

Ferner können bei der Dispergierung Feststoffpartikel (ebenso Flüssigkeit) durch Stoßwellen- oder Ultraschallenergie in einen anderen Träger dispergiert werden. Dies ermöglicht die Herstellung feinster Farbverteilung in Lösungen, vereinfacht die Herstellung von pharmazeutischen Produkten und vereinfacht auch die Aufspaltung von Zellulosefasern.

Ein vorteilhaftes Einsatzgebiet des erfindungsgemäßen Verfahrens umfaßt die Emulgierung zur Herstellung von Mischprodukten, beispielsweise Margarinerohstoff. Auch in der kosmetischen Industrie kann das Verfahren eingesetzt werden.

Das erfindungsgemäße Verfahren ist auch für eine Stoßwellen oder Ultraschallanwendung in der Textil- und Papierindustrie geeignet. Insbesondere kann eine Vorbehandlung von Zellulosefasern oder von zellulosehaltigen Stoffen mit Stoßwellen oder Ultraschall unter Verwendung der Mikrobläschen enthaltenden oder erzeugenden Mittel vorgenommen werden. Ein weiteres Einsatzgebiet ist die Entschäumung in industriellen Prozessen und in Kläranlagen. Insbesondere kann die für einen Umweltschutz bedeutungsvolle Abwasserbehandlung verbessert werden.

In vorteilhafter Weise können

1. eine Supension mit Mikrobläschen bestehend aus Mikropartikeln aus einer grenzflächenaktiven Substanz in einem flüssigen Träger oder

2. eine Suspension mit Mikrobläschen bestehend aus Mikropartikeln aus einem nicht grenzflächenaktiven Feststoff in einem flüssigen Träger oder

3. eine Suspension mit Mikrobläschen bestehend aus Mikropartikeln aus einer Mischung aus mindestens einer grenzflächenaktiven Substanz mit einem nicht grenzflächenaktiven Feststoff in einem flüssigen Träger für das Stoßwellen- oder Ultraschallarbeitsverfahren Verwendung finden.

Einige dieser erfindungsgemäß verwendeten Mittel werden in den EP-OS 122 624 und 123 235 beschrieben.

Mit Vorteil kann das verwendete Mittel Mikropartikel enthalten, die als grenzflächenaktive Substanz Lecithine, Polyoxyethylenfettsäureester, Glycerinpolyethylenglykolrizinoleat, Polyoxyethylenpolyoxypropylen-Polymere, Saccharoseester, Xyloglyceride, gesättigte oder ungesättigte (C4-C20)-Fettalkohole, gesättigte oder ungesättigte (C4-C20)-Fettsäuren oder deren Salze, Mono-, Di- und Triglyceride, Fettsäureester als Mikropartikel. Ferner ist es möglich, daß das Mittel Mikropartikel enthält, die als grenzflächenaktiven Stoff Sojaölsaccharoseglycerid oder Polyethylenglykolsorbitanmonostearat enthalten.

Mit besonderem Vorteil können als Mikropartikel Magnesiumstearat, Ascorbylpalmitat, Saccharosemonopalmitat, Saccharosemonostearat, Saccharosedistearat oder Butylstearat als grenzflächenaktive Substanzen im Mittel enthalten sein.

Die Suspension mit Mikrobläschen enthält die grenzflächenaktive Substanz in einer Konzentration von 0,001 bis 5 Gewichtsprozenten, vorzugsweise von 0,04 bis 1 Gewichtsprozent.

Als nicht grenzflächenaktive Feststoffe kann das Mittel mit Vorzug Cyclodextrine, Monosaccharide, Disaccharide, Trisaccharide, Polyole oder anorganische oder organische Salze mit einer Konzentration von 2 bis 50 Gewichtsprozenten, vorzugsweise von 9 bis 40 Gewichtsprozenten enthalten. Das Mittel kann ferner Mikropartikel enthalten, die als nicht grenzflächenaktiven Feststoff Dextrose, Maltose, Galactose, Lactose oder a-Cyclodextrin in einer Konzentration von 2 bis 5 Gewichtsprozenten, vorzugsweise 9 bis 40 Gewichtsprozenten, enthalten.

Als geeignete anorganische oder organische Salze sind Natriumchlorid, Natriumcitrat, Natriumacetat oder Natriumtartrat zu nennen.

Mit Vorteil kann das verwendete Mittel als flüssigen Träger Wasser, Elektrolytlösung, wäßrige Lösung von ein- oder mehrwertigen Alkoholen oder Polyetheralkoholen oder wäßrige Lösung eines Mono- oder Disaccharids oder Ringer-Lösung oder Tyrode-Lösung oder eine wäßrige Lösung von Maltose, Dextrose, Lactose oder Galactose enthalten. Mit besonderem Vorteil kann der flüssige Träger Glycerin, Polyethylenglycol oder Propylenglykolmethylether enthalten.

Als flüssiger Träger kann aber auch eine Kochsalzlösung in dem Mittel enthalten sein.

Überraschenderweise wurde weiter gefunden, daß ein erfindungsgemäß verwendetes Mittel, welches Mikropartikel aus Maltose, Dextrose, Lactose oder Galactose in einem flüssigen Träger der Wasser, eine physiologische Elektrolytlösung wie 0,9 %ige wäßrige Natriumchloridlösung, Ringer-Lösung oder Tyrode-Lösung oder eine wäßrige Lösung von Maltose, Dextrose, Lactose oder Galaktose sein kann, enthält, ohne Zusatz von viskositätserhöhenden Stoffen wie beispielsweise Propylenglykol eine gute Wirkungsverstärkung bei den Stoßwellen- und Ultraschallarbeitsverfahren ermöglicht.

Derartige, erfindungsgemäß verwendete Mittel werden in der EP-OS 131 540 beschrieben.

Dabei kann das erfindungsgemäß benutzte Mittel Mikropartikel aus Lactose in bis zu 25 %iger (Gewichtsprozent) wäßriger Lactose-Lösung enthalten. Insbesondere können die erfindungsgemäß verwendeten Mittel auch Mikropartikel aus Galaktose in bis zu 20 %iger wäßriger Galaktose-Lösung oder Mikropartikel aus Galaktose in Wasser enthalten.

Ferner ist vorgesehen, daß das Mittel Mikropartikel einer Mischung von Butylstearat und Galactose in Wasser oder eine Mischung von Sojaölsaccharoseglycerid und Galactose in Wasser oder Polyethylenglycolsorbitanmonostearat und Galactose in physiologischer Kochsalzlösung oder Ölsäure und Galactose in physiologischer Kochsalzlösung enthält.

Es liegt ferner im Rahmen der Erfindung, daß ein erfindungsgemäß verwendetes Mittel eine flüssige Lösung mit Mikrobläschen ist, bestehend aus der Mischung von 0,01 bis 10 Gewichtsprozenten eines Tensides oder Tensidgemisches mit einer wäßrigen oder mit Wasser mischbaren Trägerflüssigkeit und der Mischung von 0,5 bis 50 Gewichtsprozenten einer viskositätserhöhenden Substanz oder eines Substanzgemisches in einer wäßrigen oder mit Wasser mischbaren Trägerflüssigkeit, wobei beide Mischungen getrennt oder vereinigt vorliegen.

Mit besonderem Vorteil kann ein Mittel für das Stoßwellen- und Ultraschallarbeitsverfahren benutzt werden, bestehend aus einer Mischung von 0,01 bis 10 Gewichtsprozenten eines Tensides oder Tensidgemisches in einer wäßrigen oder mit Wasser mischbaren Trägerflüssigkeit, die 0,05 bis 5 Gewichtsprozente eines physiologisch verträglich carbonsauren Salzes enthält und der Mischung von 0,5 bis 50 Gewichtsprozenten einer viskositätserhöhenden Substanz oder eines Substanzgemisches mit einer wäßrigen oder mit Wasser mischbaren Trägerflüssigkeit, die dem carbonsauren Salz äquivalente Menge physiologisch verträglicher Säure enthält.

Als carbonsaures Salz findet besonders Natriumhydrogencarbonat und Kaliumhydrogencarbonat Anwendung. Als Säuren sind besonders Milchsäure, Zitronensäure und Brenztraubensäure zu nennen.

Derartig erfindungsgemäß verwendete Mittel werden in der EP-PS 0077 752 beschrieben.

Als Tenside sind sowohl ionogene als auch nichtionogene Tenside, die gleichzeitig auch viskositätserhöhend wirken können, geeignet. Als nichtionogene Tenside seien genannt: Lecithine, Lecithin-fraktionen und deren Abwandlungsprodukte, Poly-oxyethylenfettsäureester wie Polyoxyethylenfettal-koholäther, polyoxyethylierte Sorbitanfettsäure-ester, Glycerin-polyethylenglykoloxystearat, Glycer-inpolyethylenglykolrhizinoleat, ethoxylierte Sojaste-rine, ethoxylierte Rizinusöle und deren hydrierte Derivate, Cholesterol, Polyoxyethylenpolyoxypropy-len-Polymere, wobei Polyoxyethylenfettsäurestea-te und Polyoxyethylenpolyoxypropylen-Polymer mit dem Molgewicht 6800-8975, 13300 und 16250 bevorzugt sind.

Als ionogene Tenside kommen in Frage: Quarter-näre Ammoniumbase, Natriumlaurylsulfat, Natrium-dioctylsulfosuccinat.

Als viskositätserhöhende Substanzen kommen in Frage Mono- oder Polysaccharide wie Glucose, Lävulose, Galactose, Lactose, Sorbit, Mannit, Xylit, Saccharose oder Dextrane, Cyclodextrine, Hydroxyethylstärke und Polyole. Als Polyole werden verwendet Glycerin, Polyglykole, Inulin und 1,2-Propandiol. Zur Viskositäterhöhung können weiterhin benutzt werden Proteine, proteinähnliche Stoffe, Aminosäuren oder Blutersatzstoffe wie beispielsweise Plas-maproteine, Gelatine, Oxypolygelatine und Gelatine-derivate oder deren Gemische.

Die Konzentration dieser genannten Stoffe in der Lösung kann 0,5 bis 50 Gewichtsprozente betragen, wobei die Höchstkonzentration auch vom gelösten Stoff abhängt. So können beispielsweise Glucose oder Lactose mit einer Konzentration von 0,5 bis 50 Gewichtsprozenten verwendet werden, wogegen Gelatine eine bevorzugte Konzentration von 0,5 bis 2 Gewichtsprozenten hat. Die Oxypolygelatine wird bevorzugt mit einer Konzentration von 0,5 bis 10 Gewichtsprozenten eingesetzt.

Man kann auch Tenside verwenden, die gleichzeitig viskositätserhöhend wirken wie beispielsweise Polyoxyethylenpolyoxpropylen-Polymere mit dem Molekulargewicht von 4750 bis 16250.

In diesem Fall beträgt die Konzentration der Tenside mit viskositätserhöhender Wirkung 1 bis 20 Gewichtsprozenten, vorzugsweise 3 bis 10 Gewichtsprozenten. Das Tensid oder Tensidgemisch wird vorzugsweise in Gegenwart des viskositätserhöhenden Stoffes oder Stoffgemische in einer Trägerflüssigkeit gelöst.

Als Trägerflüssigkeit können Wasser oder wäßrige Lösungen, mit Wasser mischbare ein- oder mehrwertige Alkohole, Ringerlösung, Tyrodelösung oder die wäßrigen Lösungen von Natriumchlorid, Calciumchlorid, Natriumhydrogencarbonat, Natriumcitrat, Natriumacetat oder Natriumtartrat oder Salzlösungen, wie sie üblicherweise als Infusionslösungen verwendet werden, oder deren Gemische verwendet werden.

Überraschenderweise hat sich gezeigt, daß es inbesondere vorteilhaft ist, zur Zerstörung biologischer Gewe be unter Verwendung des erfindungsgemäßen Verfahrens dem Bereich des zu zerstörenden Gewebes mindestens eines der vorstehend aufgeführten Mittel zu applizieren.

Dabei ist es besonders vorteilhaft, dem Bereich des zu zerstörenden Gewebes Mikrobläschen zu applizieren, die eine koaleszente widerstandtähige Oberflächenmembran aufwiesen, in der eine Anzahl nicht toxischer und nicht antigener organische Moleküle angeordnet sind, die ein Gas einer ausgewählten Zusammensetzung enthält, wobei der Durchmesser der Mikrobläschen einen Durchmesser nicht größer als 300 Micron und nicht kleiner als 0,5 Micron ist. Insbesondere weisen die Moleküle der Oberflächenmembran einen hydrophilen und einen hydrophoben Abschnitt auf, wobei deren hydrophiler Abschnitt radial von der Mitte eines Mikrobläschen fortgerichtet ist. Mit besonderem Vorteil wird eine Oberflächenmembran für die Mikrobläschen verwendet, die aus einer gelierbaren Zusammensetzung, insbesondere Gelantine be-steht. Derartige, erfindungsgemäße verwendete Mi-krobläschen werden in der US-PS 42 76 885 beschrieben.

In der EP 0 284 802 wird eine Zerstörung von biologischen Zellen insbesondere Tumoren beschrieben, bei der die Zellen mit Metallpartikeln markiert und einer Stoßwellen und/oder Ultraschallapplikation ausgesetzt werden. Diese Mikropartikel werden durch die Ultraschall- oder Stoßwellenenergie derart beaufschlagt, daß sie schrot- oder schrapnellartig zur Zerstörung des Gewebes beitragen.

Gemäß weiterer Erfindung sollen für die Therapie von Tumorgewebe und/oder Tumorzellen oder an-dersartiger pathologischer Gewebezellen mit Stoß-wellen oder Ultraschallwellen Mittel zur Verfügung gestellt werden, die einen synagistischen Effekt ausüben.

Überraschend wurde gefunden, daß alle im vor-stehenden aufgeführten Mittel ausgezeichnete Er-gebnisse bei der Stoßwellen und Ultraschalltherapie pathologischer Gewebe, insbesondere Tumore ermöglichen.

Werden Mikrobläschen, die eine koaleszente widerstandsfähige Oberflächenmembran aufweisen, in der eine Anzahl nicht toxischer und nicht antigener organischer Moleküle angeordnet sind, verwendet, so wird in vorteilhafter Weise die Möglichkeit gegeben, zur gezielten Anwendung dieser Mikrobläschen den Durchmesser im Bereich von 300 Micron bis 0,05 Micron einzustellen. Hierdurch besteht die Möglichkeit, gezielt diese Mikrobläschen bestimmten Körperregionen zu applizieren. Durch den Durchmesser der Bläschen wird festgelegt, in welche Gewebeteile diese Bläschen eindringen können und in welche nicht. Von besonderem Vorteil ist die Verwendung von Mikrobläschen, deren Oberflächenmembran aus einer gelierbaren Zusammensetzung, insbesondere Gelatine besteht.

Ausführungsbeispiele:

Beispiel 1:
In einem Wasserbad, welches mit einem Funkenentladungsstoßwellenerzeuger versehen ist, werden in einem Probengefäß in Gelantine eingebettete Leukämiezellen eingebracht. Das Probengefäß wird dann in den zweiten Brennpunkt des Elipsoids gebracht und den Stoßwellen (250 Pulse) ausgesetzt.

Beispiel 2:
In analoger Weise werden eine gleiche Anzahl (wie in Beispiel 1 genannt) von in Gelantine eingebettete Leukämiezellen und einer Suspension aus Galactose-Mikropartikel und Mikrobläschen in einer 20%iger wäßrigen Galactoselösung in das Probengefäß eingebracht und ebenfalls 250 Pulsen von Stoßwellen ausgesetzt.

Anschließend werden mittels eines Zellzählapparates (Fluoreszensverfahren) die nach der Stoßwellenbehandlung noch vorhandenen intakten Zellen der Beispiele 1 und 2 gezählt. Dabei wurde festgestellt, daß 0% der ursprünglich vorhandenen Zellen des Beispiels 1 und 15% der Zellen des Beispiels 2 zerstört worden sind.

**Patentansprüche**

1. Arbeitsverfahren bei dem Stoßwellen oder ein Ultraschallfeld in ein Medium eingestrahlt werden, in dem eine vorbestimmte Wirkung erzielt werden soll,
dadurch gekennzeichnet, daß
1. dem Medium ein Mikrobläschen enthaltendes und/oder erzeugendes Mittel mit oder ohne Mikropartikel zugesetzt wird,
und
2. daß die Größe der Mikrobläschen und der Frequenzbereich des Ultraschallfeldes so aufeinander abgestimmt werden, daß die Resonanzfrequenz der Mikrobläschen im Frequenzbereich des Ultraschallfeldes liegt.
2. Verfahren nach Anspruch 1,
dadurch gekennzeichnet, daß
3. die Stoßwellen oder das Ultraschallfeld auf einen vorbestimmten Bereich fokussiert werden.
3. Verfahren nach Anspruch 2,
dadurch gekennzeichnet, daß
3.1 in den vorbestimmten Bereich die Stoßwellen oder das Ultraschallfeld von Erzeugern aus unterschiedlichen Richtungen eingestrahlt werden.
4. Verfahren nach Anpruch 2,
dadurch gekennzeichnet, daß
3.2 der oder die Erzeuger der Stoßwellen oder des Ultraschallfeldes um den Fokus herum verschwenkt werden.
5. Mittel zur Durchführung des Verfahrens nach mindestens einem der Ansprüche 1 - 4 gekennzeichnet durch
eine Suspension mit Mikrobläschen bestehend aus Mikropartikeln aus einer grenzflächenaktiven Substanz in einem flüssigen Träger.
6. Mittel zur Durchführung des Verfahrens nach mindestens einem der Ansprüche 1 - 4 gekennzeichnet durch
eine Suspension mit Mikrobläschen aus einem nicht grenzflächenaktiven Feststoff in einem flüssigen Träger.
7. Mittel zur Durchführung des Verfahrens nach mindestens einem der Ansprüche 1 - 4 gekennzeichnet durch
eine Suspension mit Mikrobläschen bestehend aus Mikropartikeln aus einer Mischung aus mindestens einer grenzflächenaktiven Substanz mit einem nicht grenzflächenaktiven Feststoff in einem flüssigen Träger.
8. Mittel nach einem der Ansprüche 5 oder 7, enthaltend
als grenzflächenaktive Substanz lecithine, Polyoxyethylenfettsäureester, Glycerinpolyethylenglykolrizinoleat, Polyoxyethylenpolyoxypropylen-Polymere, Saccharoseester, Xyloglyceride, gesättigte oder ungesättigte (C4-C20)-Fettalkohole, gesättigte oder ungesättigte (C4-C20)-Fettsäuren oder deren Salze, Mono-, Di- und Triglyceride, Fettsäureester, Sojaölsaccharoseglycerid oder Polyethylenglykolsorbitanmonostearat als Mikropartikel in einer Konzentration von 0,001 bis 10 Gewichtsprozenten, vorzugsweise 0,04 bis 1 Gewichtsprozent.
9. Mittel nach Anspruch 5 oder 7, enthaltend
als grenflächenaktive Substanz Magnesiumstearat, Ascorbylpalmitat, Saccharosemonopalmitat, Saccharosemonostearat, Saccharosedistearat oder Butylstearat in einer Konzentration von 0,001 bis 10 Gewichtsprozenten, vorzugsweise 0,04 bis 1 Gewichsprozent.
10. Mittel nach Anspruch 6 oder 7, enthaltend
als nicht grenzflächenaktiven Feststoff Cyclodextrine, Monosaccharide, Disaccharide, Trisaccharide, Polyole oder anorganische oder organische Salze mit einer Konzentration von 2 bis 50 Gewichtsprozenten, vorzugsweise von 9 bis 40 Gewichtsprozenten.
11. Mittel nach Anspruch 6 oder 7, enthaltend
als nicht grenzflächenaktiven Feststoff Galactose, Dextrose, Maltose, Lactose oder a-Cyclodextrin in einer Konzentration von 2 bis 50 Gewichtsprozenten, vorzugsweise von 9 bis 40 Gewichtsprozenten.
12. Mittel nach einem der Ansprüche 5 - 7, enthaltend
als flüssigen Träger, der physiologisch verträglich ist, Wasser, physiologische Electrolytlösung, wäßrige Lösung von ein- oder mehrwertigen Alkoholen oder Polyetheralkoholen oder der wäßrigen Lösung eines Mono- oder Disaccharides.

13. Mittel nach einem der Ansprüche 5 - 7, enthaltend

als flüssigen Träger Ringer-Lösung oder Tyrode-Lösung und eine wäßrige Lösung von Maltose, Dextrose, Lactose oder Galactose.

14. Mittel nach einem der Ansprüche 5 - 7, enthaltend

als flüssigen Träger eine wäßrige Lösung von Glycerin, Polyethylenglykol oder Propylenglykolmethylether.

15. Mittel nach Anspruch 7, enthaltend

Mikropartikel einer Mischung aus Butylstearat und Galactose in Wasser.

16. Mittel nach Anspruch 6, enthaltend

Mikropartikel aus Galactose in Wasser.

17. Mittel nach Anspruch 6, enthaltend

Mikropartikel aus Galactose in bis 20 %iger (Gewichtsprozent) wäßriger Galactose-Lösung.

18. Mittel nach Anspruch 5, enthaltend

Mikropartikel einer Mischung aus Polyethylenglykolsorbitanmonostearat und Galactose in physiologischer Kochsalzlösung.

19. Mittel nach Anspruch 7, enthaltend

Mikropartikel einer Mischung aus Ölsäure und Galactose in physiologischer Kochsalzlösung.

20. Mittel nach Anspruch 6, enthaltend

Mikropartikel aus Lactose in bis zu 25 %iger (Gewichtsprozent) wäßriger Lactose-Lösung.

21. Mittel zur Durchführung des Verfahrens nach mindestens einem der Ansprüche 1 - 4 gekennzeichnet durch eine flüssige Lösung mit Mikrobläschen

bestehend aus

    1. der Mischung von 0,01 bis 10 Gewichtsprozenten eines Tensides oder Tensidgemisches mit einer wäßrigen oder mit Wasser mischbaren Trägerflüssigkeit und

    2. der Mischung von 0,5 bis 50 Gewichtsprozenten einer viskositätserhöhenden Substanz oder eines Substanzgemisches in einer wäßrigen oder mit Wasser mischbaren Trägerflüssigkeit, wobei

    3. beide Mischungen getrennt oder vereinigt vorliegen.

22. Mittel nach Anspruch 21, bestehend aus der Mischung von

    1. 0,01 bis 10 Gewichtsprozenten eines Tensides oder Tensidgemisches in einer wäßrigen oder mit Wasser mischbaren Trägerflüssigkeit, die 0,05 bis 5 Gewichtsprozente eines physiologisch verträglichen carbonsauren Salzes enthält und

    2. der Mischung von 0,5 bis 50 Gewichtsprozenten einer viskositätserhöhenden Substanz oder eines Substanzgemisches mit einer wäßrigen oder mit Wasser mischbaren Trägerflüssigkeit, die die dem carbonsauren Salz äquivalente Menge physiologisch verträglicher Säure enthält.

23. Mittel nach einem der Ansprüche 21 oder 22, enthaltend

ein nichtionogenes Tensid, vorzugsweise ein Polyoxyethylenpolyoxypropylen-Polymeres, das gleichzeitig viskositätserhöhend wirkt.

24. Mittel nach Anspruch 23, enthaltend

ein Tensid, das aus Polyoxyethylenpolyoxypropylen-Polymere mit den Molekulargewicht 6800 bis 8975 oder 16250 oder 13300 oder das aus einem Polyoxyethylenfettsäureester besteht.

25. Mittel nach einem der Ansprüche 21 oder 22, enthaltend

ein Tensid, das aus Polyoxyethylenstearaten oder aus Natriumlaurylsulfat oder Natriumdioctylsulfosuccinat besteht.

26. Mittel nach Anspruch 21, enthaltend

als Trägerflüssigkeit Wasser oder mit Wasser mischbare ein- oder mehrwertige Alkohole, physiologische Elektrolytlösung oder eine Infusionslösung oder deren Gemische.

27. Mittel zur Durchführung eines Verfahrens nach einem der Ansprüche 1 - 4 dadurch gekennzeichnet,

daß die Mikrobläschen eine koaleszente widerstandsfähige Oberflächenmembran aufweisen, in der eine Anzahl nicht toxischer und nicht antigener organischer Moleküle angeordnet ist und die ein Gas ausgewählter Zusammensetzung enthält, wobei der Durchmesser der Mikrobläschen nicht größer als 300 Micron und nicht kleiner als 0,5 Micron ist.

28. Mittel nach Anspruch 27, dadurch gekennzeichnet, daß

die Moleküle der Oberflächenmembran einen hydrophilen und einen hydrophoben Abschnitt aufweisen und daß deren hydrophiler Abschnitt radial von der Mitte eines Mikrobläschens fortgerichtet ist.

29. Mittel nach einem der Ansprüche 27 oder 28, dadurch gekennzeichnet, daß

die Oberflächenmembran für die Mikrobläschen aus einer gelierbaren Zusammensetzung besteht.

30. Mittel nach Anspruch 29, dadurch gekennzeichnet, daß

die gelierbare Zusammensetzung Gelantine ist.

31. Verfahren nach mindestens einem der Ansprüche 1 - 4, mit einem Mittel nach mindestens einem der Ansprüche 5 - 30 zur Zerstörung biologischen Gewebes.

32. Verwendung eines Mittels nach mindestens einem der Ansprüche 5 - 30 zur Zerstörung von Tumorgewebe und/oder -zellen oder andersartiger pathologischer Gewebezellen mittels Stoßwellen oder Ultraschall.